# EUROPEAN PATENT SPECIFICATION

(11) **EP 1 263 781 B1**
(45) Date of publication and mention of the grant of the patent: **22.11.2006**
(21) Application number: 01911862.9
(22) Date of filing: 08.03.2001
(51) Int. Cl.: C07K 14/11, A61K 39/145, C12Q 1/68, A61P 31/14, C12N 15/44, C12N 15/62

(54) **VIRAL ANTIGEN AND VACCINE AGAINST ISAV (INFECTIOUS SALMON ANAEMIA VIRUS)**
VIRUSANTIGENE UND IMPFSTOFF GEGEN INFEKTIÖSES LACHSANÄMIEVIRUS
ANTIGENE VIRAL ET VACCIN CONTRE L'AIS (ANEMIE INFECTIEUSE DU SAUMON)

(30) Priority: 08.03.2000 GB 0005457; 14.03.2000 GB 0005960; 01.12.2000 GB 0029409
(43) Date of publication of application: 11.12.2002
(73) Proprietor: The University Court of The University of Aberdeen, Aberdeen AB24 3FX (GB)
(72) Inventor: MELVIN, William, Thomas, Aberdeen, Grampian AB15 7PQ (GB); BREEMAN, Suzanne, Ellon, AB41 8AL (GB); LABUS, Marie, Beagley, Inverurie, Grampian AB51 5QN (GB)
(74) Representative: Kremer, Simon Mark
(86) International application number: PCT/GB2001/001013
(87) International publication number: WO 2001/066569

(56) References cited:
- EP-A- 1 094 069
- WO-A-01/10469
- MJAALAND S ET AL: "GENOMIC CHARACTERIZATION OF THE VIRUS CAUSING INFECTIOUS SALMON ANEMIA IN ATLANTIC SALMON (SALMO SALAR L.): AN ORTHOMZXO-LIKE VIRUSIN A TELEOST" JOURNAL OF VIROLOGY, THE AMERICAN SOCIETY FOR MICROBIOLOGY, US, vol. 71, no. 10, October 1997 (1997-10), pages 7681-7686, XP000889868 ISSN: 0022-538X cited in the application
- SNOW ET AL: "Characterisation of the putative nucleoprotein gene of infectious salmon anaemia virus (ISAV)" VIRUS RESEARCH, vol. 74, no. 1-2, 20 February 2001 (2001-02-20), pages 111-118,

### SEQUENCE LISTING

<110> The University Court of the University of Aberdeen
   Melvin, William T
   Breeman, Suzanne
   Labus, Marie B
<120> Viral Antigen
<130> SMK5918545
<140> PCT/GB01/01013
   <141> 2001-03-08
<150> GB 0005457.7
   <151> 2000-03-08
<150> GB 0005960.0
   <151> 2000-03-14
<150> GB 0029409.0
   <151> 2000-12-01
<160> 19
<170> PatentIn Ver. 2.1
<210> 1
   <211> 1850
   <212> DNA
   <213> Infectious salmon anemia virus
<400> 1
   ttatggcgga taagggtatg acttattctt ttgatgtcag agacaacacc ttggttgtaa 60
   gaagatctac cgctactaaa agtggtataa agatctccta cagagaagat agaggtactt 120
   cacttctcca aaaggccttt gcggggacag atgatgaatt ctggctggag ctggatcaag 180
   acgtgtacgt agacaagagg attagaaagt tccttgagga agagaagatg aaggacatga 240
   gtcccagagt gtctggttct gtcgctgcag caatcgaaag gtcagtggag ttcgacaact 300
   tttcaaagga agcagctgcg aacatcgaga tgtcaggaga ggacgaggaa gaagcaggtg 360
   ggagtggcat ggttgacaac aagaggagga acaaaggggt ctccaacatg gcgtacaacc 420
   tttcactgtt cattggaatg gtcttccctg caatcaccac gttcttcagc gcaatcctgt 480
   cagaaggtga aatgagcatc tggcaaaacg ggcaggcaat catgaggatt ctcgctttgg 540
   ctgatgaaga cggaaagagg caaacgagaa caggcggaca gagagtggac atggcagatg 600
   ttaccaagct aaacgtggtg acagcaaacg ggaaagtcaa gcaggttgaa gtcaatttga 660
   atgacctgaa ggcagctttc agacagagca gacccaaaag gtcagactac aggaaagggc 720
   aaggatcaaa ggcaactgaa tcaagtattt ccaaccagtg catggctctg attatgaagt 780
   cagtgttgtc agcagaccag ctgtttgcac caggtgtgaa gatgatgagg accaatggtt 840
   tcaacgcatc atacactaca ctagcagaag gagccaacat tccaagcaag tacctaaggc 900
   acatgaggaa ctgcggagga gttgctctag atcttatggg gatgaagaga atcaagaatt 960
   caccggaagg agctaagtct aagatctttt ctatcatcca gaagaaagtc aggggaaggt 1020
   gtcgcactga ggagcaacgg ctgctgacta gtgcattgaa gatcagtgat ggcgagaaca 1080
   agttccaaag gatcatggac actctgtgca caagctttct gattgaccca cctagaacaa 1140
   ccaaatgctt cattccacct atctctagtc tcttgatgta cattcaggac ggaaattcag 1200
   tgttggcaat ggacttcatg aagaacggag aagatgcttg caggatctgc agagaggcaa 1260
   agctgaaggt tggagtgaac ggcacattta caatgtctgt ggctagaaca tgtgtagctg 1320
   tgtcaatggt tgcaacagca ttctgttcag cagatatcat cgagaatgca gttcctggct 1380
   cggaaaggta caggtcaaac atcaaggcaa acacaaccaa acctaagaag gactcaacat 1440
   acacaatcca agggctgagg ctgtccaatg tgaagtacga ggcaagacct gaaacatctc 1500
   aaagcaacac agatcgaagc tggcaagtga atgtcacaga cagtttcgga gggctagcgg 1560
   ttttcaacca aggcgctatc agggagatgc ttggagatgg aacatctgag acaacaagtg 1620
   tgaatgtcag ggctctggta aagaggatct tgaaatctgc ttcagaaaga agcgcaagag 1680
   ctgtaaaaac attcatggtt ggagaacaag ggaagtctgc aattgttatc tcaggagtgg 1740
   ggcttttctc tattgacttt gaaggggttg aggaggcaga gaggattact gacatgacac 1800
   ctgacatcga gttcgatgag gacgatgagg aggaggaaga cattgacatt            1850
<210> 2
   <211> 616
   <212> PRT
   <213> Infectious salmon anemia virus
<400> 2
   Met Ala Asp Lys Gly Met Thr Tyr Ser Phe Asp Val Arg Asp Asn Thr
   1               5                   10                  15
   Leu Val Val Arg Arg Ser Thr Ala Thr Lys Ser Gly Ile Lys Ile Ser
               20                  25                  30
   Tyr Arg Glu Asp Arg Gly Thr Ser Leu Leu Gln Lys Ala Phe Ala Gly
           35                  40                  45
   Thr Asp Asp Glu Phe Trp Leu Glu Leu Asp Gln Asp Val Tyr Val Asp
   50 55 60
   Lys Arg Ile Arg Lys Phe Leu Glu Glu Glu Lys Met Lys Asp Met Ser
   65 70 75 80
   Pro Arg Val Ser Gly Ser Val Ala Ala Ala Ile Glu Arg Ser Val Glu
   85 90 95
   Phe Asp Asn Phe Ser Lys Glu Ala Ala Ala Asn Ile Glu Met Ser Gly
   100 105 110
   Glu Asp Glu Glu Glu Ala Gly Gly Ser Gly Met Val Asp Asn Lys Arg
   115 120 125
   Arg Asn Lys Gly Val Ser Asn Met Ala Tyr Asn Leu Ser Leu Phe Ile
   130 135 140
   Gly Met Val Phe Pro Ala Ile Thr Thr Phe Phe Ser Ala Ile Leu Ser
   145 150 155 160
   Glu Gly Glu Met Ser Ile Trp Gln Asn Gly Gln Ala Ile Met Arg Ile
   165 170 175
   Leu Ala Leu Ala Asp Glu Asp Gly Lys Arg Gln Thr Arg Thr Gly Gly
   180 185 190
   Gln Arg Val Asp Met Ala Asp Val Thr Lys Leu Asn Val Val Thr Ala
   195 200 205
   Asn Gly Lys Val Lys Gln Val Glu Val Asn Leu Asn Asp Leu Lys Ala
   210 215 220
   Ala Phe Arg Gln Ser Arg Pro Lys Arg Ser Asp Tyr Arg Lys Gly Gln
   225 230 235 240
   Gly Ser Lys Ala Thr Glu Ser Ser Ile Ser Asn Gln Cys Met Ala Leu
   245 250 255
   Ile Met Lys Ser Val Leu Ser Ala Asp Gln Leu Phe Ala Pro Gly Val
   260 265 270
   Lys Met Met Arg Thr Asn Gly Phe Asn Ala Ser Tyr Thr Thr Leu Ala
   275 280 285
   Glu Gly Ala Asn Ile Pro Ser Lys Tyr Leu Arg His Met Arg Asn Cys
   290 295 300
   Gly Gly Val Ala Leu Asp Leu Met Gly Met Lys Arg Ile Lys Asn Ser
   305 310 315 320
   Pro Glu Gly Ala Lys Ser Lys Ile Phe Ser Ile Ile Gln Lys Lys Val
   325 330 335
   Arg Gly Arg Cys Arg Thr Glu Glu Gln Arg Leu Leu Thr Ser Ala Leu
   340 345 350
   Lys Ile Ser Asp Gly Glu Asn Lys Phe Gln Arg Ile Met Asp Thr Leu
   355 360 365
   Cys Thr Ser Phe Leu Ile Asp Pro Pro Arg Thr Thr Lys Cys Phe Ile
   370 375 380
   Pro Pro Ile Ser Ser Leu Leu Met Tyr Ile Gln Asp Gly Asn Ser Val
   385 390 395 400
   Leu Ala Met Asp Phe Met Lys Asn Gly Glu Asp Ala Cys Arg Ile Cys
   405 410 415
   Arg Glu Ala Lys Leu Lys Val Gly Val Asn Gly Thr Phe Thr Met Ser
   420 425 430
   Val Ala Arg Thr Cys Val Ala Val Ser Met Val Ala Thr Ala Phe Cys
   435 440 445
   Ser Ala Asp Ile Ile Glu Asn Ala Val Pro Gly Ser Glu Arg Tyr Arg
   450 455 460
   Ser Asn Ile Lys Ala Asn Thr Thr Lys Pro Lys Lys Asp Ser Thr Tyr
   465 470 475 480
   Thr Ile Gln Gly Leu Arg Leu Ser Asn Val Lys Tyr Glu Ala Arg Pro
   485 490 495
   Glu Thr Ser Gln Ser Asn Thr Asp Arg Ser Trp Gln Val Asn Val Thr
   500 505 510
   Asp Ser Phe Gly Gly Leu Ala Val Phe Asn Gln Gly Ala Ile Arg Glu
   515 520 525
   Met Leu Gly Asp Gly Thr Ser Glu Thr Thr Ser Val Asn Val Arg Ala
   530 535 540
   Leu Val Lys Arg Ile Leu Lys Ser Ala Ser Glu Arg Ser Ala Arg Ala
   545 550 555 560
   Val Lys Thr Phe Met Val Gly Glu Gln Gly Lys Ser Ala Ile Val Ile
   565 570 575
   Ser Gly Val Gly Leu Phe Ser Ile Asp Phe Glu Gly Val Glu Glu Ala
   580 585 590
   Glu Arg Ile Thr Asp Met Thr Pro Asp Ile Glu Phe Asp Glu Asp Asp
   595 600 605
   Glu Glu Glu Glu Asp Ile Asp Ile
   610 615
<210> 3
   <211> 159
   <212> PRT
   <213> Infectious salmon anemia virus
<400> 3
   Met Ala Asp Lys Gly Met Thr Tyr Ser Phe Asp Val Arg Asp Asn Thr
   1               5                   10                  15
   Leu Val Val Arg Arg Ser Thr Ala Thr Lys Ser Gly Ile Lys Ile Ser
               20                  25                  30
   Tyr Arg Glu Asp Arg Gly Thr Ser Leu Leu Gln Lys Ala Phe Ala Gly
           35                  40                  45
   Thr Asp Asp Glu Phe Trp Leu Glu Leu Asp Gln Asp Val Tyr Val Asp
       50                  55                  60
   Lys Arg Ile Arg Lys Phe Leu Glu Glu Glu Lys Met Lys Asp Met Ser
   65                  70                  75                  80
   Pro Arg Val Ser Gly Ser Val Ala Ala Ala Ile Glu Arg Ser Val Glu
                   85                  90                  95
   Phe Asp Asn Phe Ser Lys Glu Ala Ala Ala Asn Ile Glu Met Ser Gly
               100                 105                 110
   Glu Asp Glu Glu Glu Ala Gly Gly Ser Gly Met Val Asp Asn Lys Arg
           115                 120                 125
   Arg Asn Lys Gly Val Ser Asn Met Ala Tyr Asn Leu Ser Leu Phe Ile
       130                 135                 140
   Gly Met Val Phe Pro Ala Ile Thr Thr Phe Phe Ser Ala Ser Gly
   145                 150                 155
<210> 4
   <211> 148
   <212> PRT
   <213> Infectious salmon anemia virus
<400> 4
   Ser Gly Met Val Asp Asn Lys Arg Arg Asn Lys Gly Val Ser Asn Met
   1 5 10 15
   Ala Tyr Asn Leu Ser Leu Phe Ile Gly Met Val Phe Pro Ala Ile Thr
   20 25 30
   Thr Phe Phe Ser Ala Ile Leu Ser Glu Gly Glu Met Ser Ile Trp Gln
   35 40 45
   Asn Gly Gln Ala Ile Met Arg Ile Leu Ala Leu Ala Asp Glu Asp Gly
   50 55 60
   Lys Arg Gln Thr Arg Thr Gly Gly Gln Arg Val Asp Met Ala Asp Val
   65 70 75 80
   Thr Lys Leu Asn Val Val Thr Ala Asn Gly Lys Val Lys Gln Val Glu
   85 90 95
   Val Asn Leu Asn Asp Leu Lys Ala Ala Phe Arg Gln Ser Arg Pro Lys
   100 105 110
   Arg Ser Asp Tyr Arg Lys Gly Gln Gly Ser Lys Ala Thr Glu Ser Ser
   115 120 125
   Ile Ser Asn Gln Cys Met Ala Leu Ile Met Lys Ser Val Leu Ser Ala
   130 135 140
   Asp Gln Leu Phe
   145
<210> 5
   <211> 18
   <212> DNA
   <213> Artificial Sequence
<220>
   <223> Description of Artificial Sequence: Primer
<220>
   <221> misc_feature
   <222> (6, 15)
   <223> n is any nucleotide
<400> 5
   atggcngaya arggnatg 18
<210> 6
   <211> 18
   <212> DNA
   <213> Artificial Sequence
<220>
   <223> Description of Artificial Sequence: Primer
<400> 6
   rttytgccad atrctcat 18
<210> 7
   <211> 18
   <212> DNA
   <213> Artificial Sequence
<220>
   <223> Description of Artificial Sequence: Primer
<220>
   <221> misc_feature
   <222> (3, 6)
   <223> n is any nucleotide
<400> 7
   gcngcnaaya thgaratg 18
<210> 8
   <211> 18
   <212> DNA
   <213> Artificial Sequence
<220>
   <223> Description of Artificial Sequence: Primer
<220>
   <221> misc_feature
   <222> (1, 7)
   <223> n is any nucleotide
<400> 8
   nccrttngty ctcatcat 18
<210> 9
   <211> 18
   <212> DNA
   <213> Artificial Sequence
<220>
   <223> Description of Artificial Sequence: Primer
<220>
   <221> misc_feature
   <222> (3, 12)
   <223> n is any nucleotide
<400> 9
   ggncaragrg tntayatg 18
<210> 10
   <211> 18
   <212> DNA
   <213> Artificial Sequence
<220>
   <223> Description of Artificial Sequence: Primer
<400> 10
   ytcytgdatr tacatcat 18
<210> 11
   <211> 20
   <212> DNA
   <213> Artificial Sequence
<220>
   <223> Description of Artificial Sequence: Primer
<400> 11
   gtcgttgcaa ccattgacac 20
<210> 12
   <211> 17
   <212> DNA
   <213> Artificial Sequence
<220>
   <223> Description of Artificial Sequence: Primer
<400> 12
   gcaagacctg aaacatc 17
<210> 13
   <211> 20
   <212> DNA
   <213> Artificial sequence
<220>
   <223> Description of Artificial Sequence: Primer
<400> 13
   taaatgtcaa tgtcttcctc 20
<210> 14
   <211> 24
   <212> DNA
   <213> Artificial Sequence
<220>
   <223> Description of Artificial Sequence: Primer
<400> 14
   acgctcgaga gatggcggat aagg 24
<210> 15
   <211> 20
   <212> DNA
   <213> Artificial Sequence
<220>
   <223> Description of Artificial Sequence: Primer
<400> 15
   catctagatg gtctgctgac 20
<210> 16
   <211> 25
   <212> DNA
   <213> Artificial Sequence
<220>
   <223> Description of Artificial Sequence: Primer
<400> 16
   aaggatcgat ggcaactgaa tcaag 25
<210> 17
   <211> 24
   <212> DNA
   <213> Artificial Sequence
<220>
   <223> Description of Artificial Sequence: Primer
<400> 17
   tgatggcgcc ttggttgaaa accg 24
<210> 18
   <211> 21
   <212> DNA
   <213> Artificial Sequence
<220>
   <223> Description of Artificial Sequence: Primer
<400> 18
   tggctcgagc atgtgtagct g 21
<210> 19
   <211> 19
   <212> DNA
   <213> Artificial Sequence
<220>
   <223> Description of Artificial Sequence: Primer
<400> 19
   gctctagaat gtcaatgtc 19

## Description

The present invention relates to therapeutic and diagnostic compositions and methods based on a viral antigen from Infectious Salmon Anaemia (ISA) virus.

### Background Art

Infectious Salmon Anaemia (ISA) virus (ISAV) causes a severe disease affecting Atlantic salmon with typical pathological changes characterised by severe anaemia, leukopenia, ascitic fluids, hemorrhagic liver necrosis and petecchiae of the visceria (Krossøy, et al., 1999).

The disease is caused by an enveloped virus, with a diameter of approximately 100nm, budding from endothelial cells lining the heart and blood vessels, as well as from polymorphonuclear leukocytes (Hovland, et al., 1994; Nyland et al., 1996).

The virus has a negative stranded RNA genome consisting of 8 segments ranging from 1- 2.3 kbp in size (total 14.5 kbp) which has led to a description of ISAV as orthomyxovirus-like (Mjaaland et al., 1997). This relationship is also supported by recent work characterising the biochemical, physiochemical and morphological properties of the ISAV compared to influenza virus (Falk et al., 1997).

In recent years, ISAV has been detected in samples from farmed Atlantic salmon on the Canadian east coast and in Scotland (Krossøy, et al., 1999). It has also been reported in the Faroes. It can be devastating (mortality 15-100%) - however existing control measures are based on minimized exposure e.g. by culling of infected fish and sanitary measures. There are few vaccines currently available.

One vaccine, originating from Microtek\Bayotek International Ltd, Canada, is based on inactivated virus.

A vaccine from Aquahealth Ltd, Canada, has been given limited permission under autologous licence for use in infected farms in Canada (Aquahealth, Product News). Jones *et al.* of Aquahealth Ltd (1999, The Bulletin of the European Association of Fish Pathologists 19(3), 98-101) report the use of a vaccine based on antigens isolated from cultured virus.

The sequence data available for this virus has been limited to date. Sequences reported as coming from ISAV are:
- A protein said to be the haemagluttinin protein from segment 7 (EMBL accession number AF220607).
- Two ORFs encoding non-structural proteins within segment 8 (Mjaaland et al., 1997).
- The PB1 gene from segment 2 (Krossøy, et al., 1999).

EP 1 094 069 (Akzo) discusses the SP-1 protein of ISAV and uses thereof. WO 01/10469 (Aquahealth) discusses various peptides and nucleic acids for use as vaccines against ISAV. In addition, WO 00/72878 (Genomar AS) discusses ISAV vaccines apparently based on certain proteins and nucleic acids. A 1173 nucleotide sequence (391 amino acids) is presented. All of these documents were published after the priority dates claimed herein.

### Disclosure of invention

The present inventors have cloned and expressed an antigen from ISA which may be used advantageously in the treatment or diagnosis of ISA. The antigen was cloned from RNA segment 3 of ISAV and is believed to be a major cell culture antigen of the virus, which may be a nucleoprotein (NP). The nucleoprotein of ISAV is a required component for viral expression, along with PB1, PB2, PA and the viral RNA, and may therefore be an important target for vaccines and diagnostics (Gammelin et al, 1990, Virology 170: pp 71-80). Hereinafter the antigen is referred to as the 'European ISAV antigen', or 'ISAV antigen'.

An antigen which may correspond to that disclosed herein may be the subject of a paper (Snow & Cunningham (2001) Virus Research 74 (1-2), pp. 111-118) published after the priority dates claimed herein. This is particularly concerned with the evolutionary position of ISAV within *Orthomyxoviridae.*

As discussed in more detail below, the peptide sequence of the ISAV antigen can be used as the basis of vaccines.

The complete sequence, or portions thereof, can be expressed in heterologous systems, such as E.coli, yeast, mammalian cells or piscine cells, and the protein product utilised for vaccination and other purposes such as the production of antibodies, for example for the development of immunoassays. Synthetic peptides derived from this sequence and other agents generated using this information can also be used for vaccine and other purposes, such as the generation of antibodies.

The use of this sequence for vaccine and other purposes will be of principal value in the application to the European strain of ISAV. However, the substances derived from this sequence information also have utility for related virus types.

Some of these aspects will now be discussed in more detail.

### ISAV antigen polypeptides

In a first aspect of the present invention there is disclosed a polypeptide consisting of the amino acid sequence of the ISAV antigen shown as Seq. ID No. 2.

As shown in Seq. ID No. 2, this antigen contains 616 amino acids.

Another preferred embodiment of the invention may be based on the antigen shown in Seq. ID No. 3 (corresponds to residues 1-156 of Seq. ID No. 2 i.e. 156 amino acids, plus a further three amino acids at the C-terminus).

Another preferred embodiment of the invention may be based on the antigen shown in Seq. ID No. 4 (corresponds to residues 121-268 of Seq. ID No. 2 i.e. 148 amino acids).

Any such polypeptide may be referred to as an ISAV antigen polypeptide hereinafter.

Polypeptides and nucleic acids (below) according to the present invention may be provided isolated and/or purified from their natural environment, in substantially pure or homogeneous form, or free or substantially free of other proteins or nucleic acids of the species of origin. Where used herein, the term "isolated" encompasses all of these possibilities.

### Recombinant production of ISAV antigen polypeptides

The polypeptides of the aspects discussed herein are preferably produced by recombinant techniques in a suitable host using nucleic acids disclosed herein.

Generally speaking, those skilled in the art are well able to construct vectors and design protocols for recombinant gene expression. Suitable vectors can be chosen or constructed, containing appropriate regulatory sequences, including promoter sequences (see below), terminator fragments, polyadenylation sequences, enhancer sequences, marker genes, signal sequences and other sequences as appropriate. For further details see, for example, *Molecular Cloning: a Laboratory Manual:* 2nd edition, Sambrook *et al,* 1989, Cold Spring Harbor Laboratory Press (or later editions of this work). Many known techniques and protocols for manipulation of nucleic acid, for example in preparation of nucleic acid constructs, mutagenesis (see above discussion in respect of variants), sequencing, introduction of DNA into cells and gene expression, and analysis of proteins, are described in detail in *Current Protocols in Molecular Biology,* Second Edition, Ausubel et al. eds., John Wiley & Sons, 1992. The disclosures of Sambrook et al. and Ausubel et al. are incorporated herein by reference.

In one aspect of the present invention, the nucleic acid encoding an ISAV antigen described above is in the form of an expression vector.

Preferably the nucleic acid in the vector is under the control of, and operably linked to, an appropriate promoter or other regulatory elements for transcription in a heterologous host cell such as E.coli, yeast, mammalian cells or piscine cells. The vector may be a bi-functional expression vector which functions in multiple hosts. In the case of cDNA this may be under the control of an appropriate promoter or other regulatory elements for expression in the host cell.

In a further aspect of the invention, there is disclosed a host cell containing or transformed with a heterologous construct according to the present invention, especially a microbial cell. The term "heterologous" is used broadly in this aspect to indicate that the ISAV antigen gene/sequence of nucleotides in question have been introduced into the host cell, or a progenitor cell thereof, using genetic engineering, i.e. by human intervention. Nucleic acid heterologous to the host cell will be non-naturally occurring in the host cell type.

A preferred host cell is *Pichia pastoris.*

Preferably, the expressed polypeptide is secreted from the host cell and the vector preferably includes a signal sequence to direct the protein so that it is secreted from the cell.

### Preparation of polypeptides

The polypeptide may be partially purified before being used as a vaccine. Where the polypeptide is secreted from the host cell, the cells may be separated from the media by centrifugation, the cells being pelleted and the media being the supernatant. The polypeptide may be partially purified from this supernatant.

Vaccine compositions of the present invention may be provided by expressing a polypeptide as described herein in a suitable host, partially purifying the expressed polypeptide from the host.

The method may further comprise admixing the partially purified polypeptide with another component, such as another polypeptide and/or an adjuvant, diluent or excipient.

### Fusions and conjugates

The ISAV antigen polypeptide may be linked to a suitable carrier to enhance immunogenicity. Suitable carriers include bovine serum albumin, keyhole limpet haemocyanin etc.

### Vaccines

A vaccine of the present invention may comprise, consist of, or consist essentially of an ISAV antigen polypeptide as described above.

A vaccine according to the present invention may comprise an ISAV antigen polypeptide as described above plus a further ISAV polypeptide. Also the ISAV antigen polypeptide may be included within a multivalent vaccine which includes antigens against other diseases of fish.

In addition to the polypeptides, the vaccine composition may further comprise a pharmologically acceptable diluent, buffer, adjuvant, or excipient, or combination of these. Such materials should be non-toxic and should not interfere with the efficacy of the active ingredient. The precise nature of the carrier or other material will depend on the route of administration, which may be oral, or by injection, e.g. intravenous.

Suitable materials are well known to the person skilled in the art. Examples include; water, saline, buffered saline, fish oil with an emulsifier (e.g. a lecithin), light mineral oil. Immersion vaccines will tend to be an aqueous suspension. A preferred adjuvant is Montanide ISA 711 adjuvant.

The polypeptides described herein may also be used in the manufacture of a vaccine or other medicament for treatment of, or having prophylactic effect against ISAV.

### Modes of administration

A vaccine composition may be administered orally or by injection. A vaccine composition may be administered alone or in combination with other treatments, either simultaneously or sequentially. A vaccine composition may be administered as a course of a number of discrete doses over a period of time. For example it may be administered over a period of around fourteen days.

The vaccine of the present invention may be administered via any suitable route, including injection into the bloodstream and oral administration. Preferred doses range from 15 to 150 µg per fish, more preferably 70 to 125 µg per fish. Most preferred is a dose of 100µg per fish e.g. dissolved in 10-100 µl.

Vaccination may be repeated at daily, twice-weekly, weekly or monthly intervals. For example a boost vaccination may be administered after the initial dose. For example a boost may be administered at around fourteen weeks after the vaccination. The initial vaccination and any boost may be carried out using the same or different modes of administration. For example, the initial may be by injection and the boost may be by oral administration. A preferred regime includes a first vaccination by injection, followed by (14 weeks post challenge) a two week course of orally administered boost vaccine.

Other suitable adjuvants, carriers etc., and modes of administration may be found by referring to Gudding et al (1999) Veterinary immunology and Immunopathology 72, 203-212; Dunn et al (1990), Aquacultural Engineering 9, 23-32; Ellis (1995) Fish pathology 30, 293-300.

### Methods of diagnosis

The polypeptides described herein may also be used in methods of diagnosis for ISAV. Such an assay could be by ELISA, or other technique as would be understood by the person skilled in the art.

### Figures and Sequence Annexes

Figure 1: Example of PCR amplification of ISAV sequence. Lane M: øx174 molecular weight markers; NC: Negative control; ISAV: PCR reaction using primer set 2.
Figure 2: 15 % Polyacrylamide gel stained with Coomassie blue to visualise the expressed ISAV protein sampled at various time points after expression.
   Seq. ID No. 1 : Sequence of the ISAV antigen gene from the Scottish strain of ISAV
   Seq. ID No. 2 : Predicted amino acid sequence of the antigen gene from the Scottish strain of ISAV
   Seq. ID No. 3 : first portion of amino acid sequence of the Scottish strain of ISAV
   Seq. ID No. 4 : second portion of amino acid sequence of the Scottish strain of ISAV

### Examples

### Example 1 - obtaining the sequence of the European ISAV antigen

### PCR primers

PCR primers were designed as follows;
ISAfor1, 5'ATGGCNGAYAARGGNATG3'; ISArev1, 5' RTTYTGCCADATRCTCAT3'; ISAfor2, 5'GCNGCNAAYATHGARATG3'; ISArev2, 5'NCCRTTNGTYCTCATCAT3'; ISAfor3, 5'GGNCARAGRGTNTAYATG; ISArev3, 5'YTCYTGDATRTACATCAT3'; or 5'GTCGTTGCAACCATTGACAC3'; ISAfor4, 5'GCAAGACCTGAAACATC3'; ISArev4, 5' TAAATGTCAATGTCTTCCTC3'.

### cDNA synthesis

Tissue culture supernatant was obtained from CHSE cells infected with a Scottish isolate of ISAV. RNA was prepared using a Qiamp Viral RNA minikit (Qiagen, West Sussex, UK) under manufacturer's instructions. cDNA was prepared using 10µl of RNA using the Riboclone cDNA synthesis system (Promega, Southampton, UK) under manufacturer's instructions.

### PCR amplification of ISAV sequence

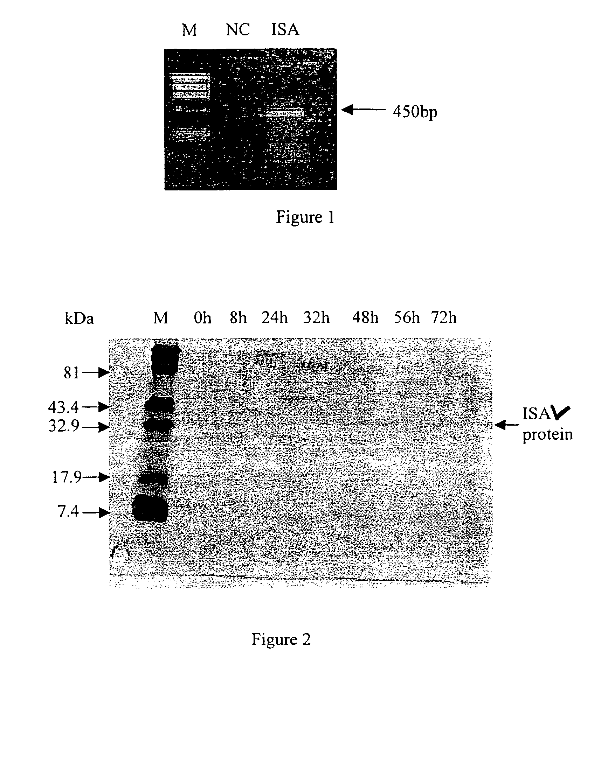

PCR was carried out using 1µl of the cDNA prepared above and 100pmol of each PCR primer in 45 µl of ReddyMix PCR Master Mix (ABgene, Surrey, UK). Amplification was carried out in a Perkin Elmer Thermal Cycler using the following cycling parameters; 94°C for 30s, 42°C for 30s, 72°C for 60s for 30 cycles, followed by a final extension step at 72°C for 10 min. A 5µl aliquot of each PCR reaction was electrophoresed through a 1% agarose gel containing 0.5µg/ml ethidium bromide. An example of one of the PCR products is shown (Figure 1).

### DNA sequence analysis

PCR products were purified using Wizard PCR preps DNA purification system (Promega, Southampton, UK) under manufacturer's instructions. DNA sequencing was carried out using the Applied Biosystems Model 373A Automated DNA sequencer according to the manufacturer's instructions with PCR products used in each PCR reaction at a concentration of 2.5pmol.

The ISAV DNA sequences were compiled and the sequence obtained translated using the GWGCG software.

### Example 2 - Expression of ISAV protein

The system used was vector pPICZα in the methylotrophic yeast Pichia *pastoris.* The procedures are described in the technical manual 'EasySelect^{™} Pichia Expression Kit' obtainable from Invitrogen Corporation, 1600 Faraday Avenue, Carlsbad, CA 92008.

### Isolation of ISAV coding regions

PCR primers were designed to allow the amplification of 3 overlapping fragments of the coding region of the ISAV gene, while introducing enzyme restriction sites to allow the subsequent cloning into the *Pichia pastoris* expression vector, pPICZαC as follows: The restriction enzyme sites are indicated by underlining.

PCR was carried out using 1µl of ISAV cDNA, 25pmoles each PCR primer in 45µl of PCR master mix containing 2.5mM MgCl₂ (ABgene). Cycling was carried out in a Perkin Elmer Thermocycler using the following cycling parameters; 30 cycles of 30s at 94°C, 30s at 42°C, 1min at 72°C and a final incubation of 10min at 72°C. A 10µl aliquot of the resultant PCR reactions were electrophoretically separated through a 1.5% agarose gel containing 0.5µg/ml ethidium bromide. If amplification of the ISAV antigen gene was successful, the DNA from the remainder of each PCR reaction was purified using the PCR clean up kit from Promega under manufacturer's instructions.

### Preparation of recombinant pPICZαC plasmids containing ISA coding regions.

The purified ISAV PCR products underwent restriction enzyme digestion to facilitate cloning into the Pichia pastoris expression vector pPICZαC. The ISAV segment 1 and segment 3 PCR products were digested using the restriction endonucleases *XhoI* and *XbaI,* the segment 2 PCR products were digested using *ClaI* and *SacII.* Restriction digestions were set up by combining the following components; 30µl purified PCR product, 4µl restriction enzyme buffer, 4µl acetylated BSA (1mg/ml) and 1µl of each restriction enzyme. The digestions were incubated at 37°C for 90min. In addition, pPICZαC plasmid was also digested using the same enzymes to allow each ISAV PCR product to be cloned. Plasmid restriction digestions were set up by combining the following components; 1µg plasmid DNA, 1µl restriction enzyme buffer, 1µl acetylated BSA (1mg/ml), 1µl each restriction enzyme and 5µl distilled water. The digestions were incubated at 37°C for 90 min. Following the incubation period, the digested DNA was purified from each sample by phenol/chloroform extraction and ethanol precipitation at -80°C for 20 min. The DNA was pelleted by centrifugation at 13,000rpm for 15 min, the pellets air dried and resuspended in 10µl distilled water. Ligations were prepared by combining the following components; 5µl digested ISAV PCR product, 1µl digested pPICZαC plasmid, 1µl 10x ligase buffer, 2µl distilled water and 1µl T4 DNA ligase. The ligations were incubated overnight at 4°C.

The following day, a 5µl aliquot of each ligation mix was used to transform electrocompetent E. coli TOP10F' cells (Invitrogen) according to the manufacturer's instructions. Following cell recovery, aliquots of the transformed cells were plated onto LB agar plates containing 25µg/ml zeocin and the plates incubated, inverted, overnight at 37°C. Each resultant colony was used to inoculate 5ml of LB medium containing 25µg/ml zeocin which was subsequently incubated overnight at 37°C with vigorous aeration. Recombinant pPICZαC plasmid was prepared from 1ml of each overnight culture using the 5'-3' plasmid preparation kit. The remaining culture was used to prepare glycerol stocks which were stored at -80°C. Recombinant plasmids were screened for the presence of a insert by restriction digestion with either *XhoI* and *XbaI* (segments 1 and 3) or *ClaI* and *SacII* (segment 2) using the protocol outlined previously. Digestions were electrophoretically separated through a 1.5% agarose gel containing 0.5µg/ml ethidium bromide. Plasmids which contained the correct sized inserts were further analysed by automated DNA sequencing.

### Preparation of Pichia pastoris cell lines expressing ISAV.

Recombinant pPICZαC plasmids which had been shown to contain segments of the ISAV antigen gene by restriction digestion analysis and DNA sequence analysis were prepared for transformation into Pichia *pastoris* strain GS115 as outlined below. Large scale plasmid preparations of each recombinant pPICZαC plasmid were carried out using the 5'-3' plasmid preparation kit under manufacturer's instructions. Approximately 10µg of each plasmid was prepared. The recombinant plasmids were linearised prior to their transformation into *P. pastoris* GS115 using the restriction enzyme *SacI* by combining the following components: 5µg recombinant plasmid (40µl approximately), 6µl 10x restriction enzyme buffer, 6µl acetylated BSA(1mg/ml), 6µl distilled water and 2µl *SacI.* The digestions were incubated at 37°C for 90 min. Following this incubation the digested plasmids were purified by phenol/chloroform extraction and ethanol precipitation at -80°C for 20 min. The linearised plasmids were resuspended in 10µl distilled water.

Electrocompetent *Pichia pastoris* GS115 cells were prepared as outlined below. A single colony of GS115 was used to inoculate 5ml of YPD medium which was then incubated overnight at 30°C with vigorous aeration. The following day 0.5ml of this overnight culture was used to inoculate 500ml of fresh YPD medium which was grown overnight as before. The cells were pelleted at 1500g for 5 min at 4°C and the pellet resuspended in 500ml of ice-cold sterile, distilled water. The cells were pelleted as before and resuspended in 250ml of ice-cold sterile, distilled water. The cells were pelleted again and resuspended in 20ml of ice-cold sterile 1M sorbitol, pelleted for the last time and resuspended in 1ml of ice-cold 1M sorbitol. The cells were used immediately. 80µl of the electrocompetent cells were mixed with 10µl linearised plasmid described above, and transferred to an ice-cold 0.2cm electroporation cuvette. The cuvette was incubated on ice for 5min. The cell and DNA mix was pulsed in a Bio-Rad Gene Pulser with a charging voltage of 1500V, a capacitance of 25µF and a resistance of 200Ω. 1ml of ice-cold 1M sorbitol was added and the contents transferred to a sterile 15ml tube. The cells were allowed to recover by incubation at 30°C for 1-2h without shaking. Aliquots of the cells were plated onto YPDS plates containing 100µg/ml zeocin. The plates were incubated for 2-3 days at 30°C until colonies formed.

Small scale expresssions were carried out using the recombinant Pichia pastoris GS115 colonies in order to screen for expression of the ISAV antigen protein in these cell lines.

Each colony was used to inoculate 25ml of MGYH medium in a 250ml flask. The cultures were incubated overnight at 30°C with vigorous aeration. The following day the cells were harvested at 1500g for 5 min at room temperature and the cell pellet resuspended in 100ml of MMH to induce protein expression. The culture was incubated in a 11 flask at 30°C with vigorous aeration with 100% methanol being added to a final concentration of 0.5% every 24h. 1ml aliquots were removed at the following time points; 0, 8, 24, 32, 48, 56 and 72 hours, and the cells were pelleted and the supernatant transferred to a fresh tube. Both cell pellet and supernatant were stored at -80°C until all time point samples had been collected.

The samples collected during the small scale expression were analysed by SDS-PAGE through a 15% acrylamide gel. Two identical gels were run for each sample, one of which was stained with Coomassie Brilliant blue to visualise the proteins and the other was western blotted onto nitrocellulose and immunoblotted with a specific anti-ISAV monoclonal antibody. This allowed the optimum expression period to be determined for each cell line.

Glycerol stocks were prepared for each cell line which showed good expression of ISAV antigen. These were stored at -80°C.

An example of the expression of segment ISA2 is shown in Figure 2. The greatest expression was found at 72h .

### Example 3 - dose response in salmon

Atlantic salmon, *Salmo salar,* for use in these experiments are reared at the Fish Cultivation Unit of the Marine Laboratory, Aultbea, Wester Ross, Scotland. Prior to all experimental procedures the fish are anaesthetised using ethyl-4-amino benzoate (Benzocaine, BDH Chemicals, Poole, Dorset, UK).

All experiments are carried out in one metre tanks containing 350 litres of fresh water, supplied with ca 10 litres per minute per tank. Fish were fed (Mainstream diets, BP Nutrition) daily to satiation.

### Immunisation.

This is based on the challenge method of Amend (1981, "Potency of fish vaccines"; Developments in Bioloigical Standardisation 49, 447-454). Five doses are used containing 10, 35, 70, 100 and 150 µg of ISAV polypeptide. The vaccine is diluted in PBS and mixed with the adjuvant (Montanide ISA 711®, Sepic) at a ratio of 30:70, respectively. Fish are i.p. injected with 0.1 ml vaccine. Fish injected with PBS plus adjuvant (ratio 30:70) are used as control. During the experimental period fish are kept between 12°C and 14°C for at least 8 weeks.

### Challenge.

Fish are challenged by immersion using 1000 infectious virus particles per ml of water. This results in a 70% mortality in the controls after 6 weeks.

### Example 4 - ISAV enzyme-linked immunobsorbant assay (ELISA)

ISAV PEG precipitated virus is diluted with 0.05 M carbonate-bicarbonate buffer, pH 9.6, to give 5 x 10⁷TCID₅₀/ml and used (100 µl) to coat individual wells (Immulon 4 HBX, Dynex Technologies Inc, USA). The coated plates are incubated at 4°C for 48 h, washed in phosphate-buffered saline (PBS) containing 0.05% Tween-20 (PBS-Tween), blocked with 5% non fat dry milk in PBS-Tween for 1 h at 37°C, washed with PBS-Tween and stored at -80°C until used.

Subsequent dilutions and washing between incubations are carried out in PBS-Tween. Salmon antisera are serially two-fold diluted (1:60 to 1:1920) and incubated in duplicate at 4°C overnight. A pool of sera from control fish is used as a negative control. A positive control is used in all plates. Incubation with PBS-Tween is used as a blank. After washing, Mouse anti-salmon Ig (4C10) diluted 1:8 was incubated for 2 h at room temperature. Horseradish peroxidase conjugate goat anti-mouse IgG (Sigma) diluted 1:1000 is incubated 1 h at room temperature.

Tetramethylbenzidine (TMB, Sigma), 100 µl/well, was added as substrate and incubated for 30 min at room temperature. Plates were read spectrophotometrically at 630 nm using an ELISA reader (DIAS, Dynatech Laboratories).

### Example 5 - identification based on nucleic acids

Material from ISAV infected and non-infected salmon is homogenised and RNA extraction carried out in accordance with commonly known biotechnological methods. Reverse transcription and PCR reactions were performed as described by the manufacturer using primers described above.

### Example 6 - ISAV serum neutralisation assay

The protective effect of vaccination with the ISAV antigen vaccine is demonstrated as follows. Heat-inactivated serum samples from vaccinated fish are prepared by serial dilution in E-MEM + 2% foetal bovine serum (FBS) and mixed with live ISA virus in 96-well microplates to a final concentration of 500 TCID₅₀ per 100 µl well. Following incubation for 1 h at room temperature, 50 µl aliquots of these samples containing virus and plasma at appropriate concentrations are applied to wells containing confluent chinook salmon embryo (CHSE-214) cells in 75 µl E-MEM +10 % FBS. Controls are prepared by substitution of pooled normal salmon serum or omission of virus as appropriate. All cultures are incubated for 7 days at 15 °C, and then virus induced cell lysis was determined by measurement of absorbance on the microplate-format spectrophotometer.

### References:

Falk, K., Namork, E., Rimstad, E., Mjaaland, S & Dannevig, B.H. (1997). Characterisation of Infectious Salmon Anemia virus, an orthomyxo-like virus isolated from Atlantic salmon (*Salmo salar* L). *Journal of Virology,* 71, 9016-9023.

Hovland, T., Nylund, A., Watanabe, K & Endresen, C. (1994). Observation of Infectious Salmon Anemia virus in Atlantic salmon, *Salmo salar* L. *Journal of Fish Diseases,* 17, 291-296.

Krossøy, B., Hordvik, I., Nilsen, F., Nylund, A & Endresen C. (1999). The putative polymerase sequence of Infectious Salmon Anemia virus suggests a new genus within the *Orthomyxoviridae. Journal of Virology,* 73(3), 2136-2142.

Mjaaland, S., Rimstad, E., Falk, K & Dannevig, B.H. (1997). Genomic characterisation of the virus causing Infectious Salmon Anemia in Atlantic salmon (*Salmo salar* L): an orthomyxo-like virus in a teleost. *Journal of Virology,* 71(10), 7681-7686.

Nylund, A., Kroossoy, B., Watanabe, K & Holm, J.A. (1996). Target cells for the ISA virus in Atlantic salmon (*Salmo salar* L). *Bulletin of the European Association of Fish Pathologists,* 16, 68-72.

### Seq. ID No. 1 : Sequence of the ISAV antigen gene from the Scottish strain of ISAV

### Seq. ID No. 2 : Predicted amino acid sequence of the antigen gene from the Scottish strain of ISAV

### Seq. ID No. 3 : portion of amino acid sequence of the Scottish strain of ISAV

### Seq. ID No. 4: portion of amino acid sequence of the Scottish strain of ISAV

## Claims

1. An isolated Infections Salmon Anaemia Virus (ISAV) antigen polypeptide consisting of the European ISAV antigen amino acid sequence shown in Seq. ID No. 2, 3 or 4.

2. An expression vector encoding the ISAV antigen polypeptide of claim 1.

3. A method of producing the ISAV antigen polypeptide of claim 1 which method comprises expressing the vector of claim 2 in a suitable host cell.

4. A method as claimed in claim 3 wherein the host cell is selected from *E. coli,* yeast, mammalian cells or piscine cells.

5. A method as claimed in claim 4 wherein the host cell is *Pichia pastoris.*

6. A vaccine comprising the ISAV antigen polypeptide of claim 1.

7. The isolated ISAV antigen polypeptide of claim 1 for use as a vaccine against infectious salmon anaemia.

8. Use of an ISAV antigen polypeptide as claimed in claim 1 in an immunoassay.

9. A nucleic acid primer selected from any of the following:
ISAfor1, ATGGCNGAYAARGGNATG
ISArev1, RTTYTGCCADATRCTCAT
ISAfor2, GCNGCNAAYATHGARATG
ISArev2, NCCRTTNGTYCTCATCAT
ISAfor3, GGNCARAGRGTNTAYATG
ISArev3A, YTCYTGDATRTACATCAT
ISA1 forward primer, ACGCTCGAGAGATGGCGGATAAGG
ISA1 reverse primer, CATCTAGATGGTCTGCTGAC
ISA2 forward primer, AAGGATCGATGGCAACTGAATCAAG
ISA2 reverse primer, TGATGGCGCCTTGGTTGAAAACCG
ISA3 forward primer, TGGCTCGAGCATGTGTAGCTG
ISA3 reverse primer, GCTCTAGAATGTCAATGTC

10. Use of a primer as claimed in claim 9 to amplify an ISAV nucleic acid sequence.

## Patentansprüche

1. Isoliertes ISAV- (Virus der Infektiösen Anämie der Lachse) Antigenpolypeptid, bestehend aus der in Seq.-ID Nr. 2, 3 oder 4 dargestellten europäischen ISAV-Antigen-Aminosäuresequenz.

2. Expressionsvektor, der für ein ISAV-Antigenpolypeptid nach Anspruch 1 kodiert.

3. Verfahren zur Herstellung eines ISAV-Antigenpolypeptids nach Anspruch 1, wobei das Verfahren das Exprimieren eines Vektors nach Anspruch 2 in einer geeigneten Wirtszelle umfasst.

4. Verfahren nach Anspruch 3, worin die Wirtszelle aus E. coli, Hefe, Säugetierzellen oder Fischzellen ausgewählt ist.

5. Verfahren nach Anspruch 4, worin die Wirtszelle Pichia pastoris ist.

6. Vakzine, umfassend ein ISAV-Antigenpolypeptid nach Anspruch 1.

7. Isoliertes ISAV-Antigenpolypeptid nach Anspruch 1 zur Verwendung als Vakzine gegen Infektiöse Anämie der Lachse.

8. Verwendung eines ISAV-Antigenpolypeptids nach Anspruch 1 in einem Immuntest.

9. Nucleinsäure-Primer, aus Folgenden ausgewählt:
ISAvor1, ATGGCNGAYAARGGNATG
ISArück1, RTTYTGCCADATRCTCAT
ISAvor2, GCNGCNAAYATHGARATG
ISArück2, NCCRTTNGTYCTCATCAT
ISAvor3, GGNCARAGRGTNTAYATG
ISArück3A, YTCYTGDATRTACATCAT
ISA1-Vorwärts-Primer, ACGCTCGAGAGATGGCGGATAAGG
ISA1-Rückwärts-Primer, CATCTAGATGGTCTGCTGAC
ISA2-Vorwärts-Primer, AAGGATCGATGGCAACTGAATCAAG
ISA2-Rückwärts-Primer, TGATGGCGCCTTGGTTGAAAACCG
ISA3-Vorwärts-Primer, TGGCTCGAGCATGTGTAGCTG
ISA3-Rückwärts-Primer, GCTCTAGAATGTCAATGTC.

10. Verwendung eine Primers nach Anspruch 9 zur Amplifikation einer ISAV-Nucleinsäuresequenz.

## Revendications

1. Polypeptide antigénique du virus de l'anémie infectieuse du saumon ( VAIS) isolé se composant de la séquence d'acides aminés antigénique du VAIS européen présentée dans la SEQ ID N° 2, 3 ou 4.

2. Vecteur d'expression codant pour le polypeptide antigénique du VAIS de la revendication 1.

3. Procédé de production du polypeptide antigénique du VAIS de la revendication 1, procédé qui comprend l'expression du vecteur de la revendication 2 dans une cellule hôte appropriée.

4. Procédé selon la revendication 3 dans lequel la cellule hôte est choisie parmi *E. coli,* des levures, des cellules de mammifère ou des cellules de poisson.

5. Procédé selon la revendication 4 dans lequel la cellule hôte est *Pichia pastoris.*

6. Vaccin contenant le polypeptide antigénique du VAIS de la revendication 1.

7. Polypeptide antigénique du VAIS isolé de la revendication 1 destiné à être utilisé en guise de vaccin contre l'anémie infectieuse du saumon.

8. Utilisation d'un polypeptide antigénique du VAIS selon la revendication 1 dans un dosage immunologique.

9. Amorce d'acide nucléique choisie parmi l'une quelconque des suivantes:
ISAfor1, ATGGCNGAYAARGGNATG
ISArev1, RTTYTGCCADATRCTCAT
ISAfor2, GCNGCNAAYATHGARATG
ISArev2, NCCRTTNGTYCTCATCAT
ISAfor3, GGNCARAGRGTNTAYATG
ISArev3A, YTCYTGDATRTACATCAT
Amorce sens ISA1, ACGCTCGAGAGATGGCGGATAAGG
Amorce antisens ISA1, CATCTAGATGGTCTGCTGAC
Amorce sens ISA2, AAGGATCGATGGCAACTGAATCAAG
Amorce antisens ISA2, TGATGGCGCCTTGGTTGAAAACCG
Amorce sens ISA3, TGGCTCGAGCATGTGTAGCTG
Amorce antisens ISA3, GCTCTAGAATGTCAATGTC

10. Utilisation d'une amorce selon la revendication 9 dans l'amplification d'une séquence d'acide nucléique du VAIS.
